(19) Europäisches Patentamt
European Patent Office
Office européen des brevets

(11) **EP 4 077 267 B1**

(12) **EUROPEAN PATENT SPECIFICATION**

(45) Date of publication and mention
of the grant of the patent:
**27.11.2024  Bulletin 2024/48**

(21) Application number: **20842393.9**

(22) Date of filing: **16.12.2020**

(51) International Patent Classification (IPC):
**C07C 67/60** *(2006.01)*     **C07C 69/82** *(2006.01)*
**C07D 317/12** *(2006.01)*     **C08J 11/04** *(2006.01)*
**C08J 11/10** *(2006.01)*

(52) Cooperative Patent Classification (CPC):
(C-Sets available)
**C07C 69/82; C07C 67/60; C07D 317/12;
C08J 11/10; C08J 11/24;** C08J 2367/02;
Y02P 20/582; Y02W 30/62          (Cont.)

(86) International application number:
**PCT/US2020/065257**

(87) International publication number:
**WO 2021/126939 (24.06.2021 Gazette 2021/25)**

(54) **METHOD FOR PRODUCING DIMETHYL TEREPHTHALATE FROM POLYESTER METHANOLYSIS DEPOLYMERIZATION SYSTEMS**

VERFAHREN ZUR HERSTELLUNG VON DIMETHYLTEREPHTHALAT AUS POLYESTERMETHANOLYSE-DEPOLYMERISATIONSSYSTEMEN

PROCÉDÉ DE PRODUCTION DE TÉRÉPHTALATE DE DIMÉTHYLE À PARTIR DE SYSTÈMES DE DÉPOLYMÉRISATION DE POLYESTER MÉTHANOLYSE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **19.12.2019   US 201962950611 P**

(43) Date of publication of application:
**26.10.2022   Bulletin 2022/43**

(73) Proprietor: **Eastman Chemical Company
Kingsport, TN 37660 (US)**

(72) Inventors:
• **LIN, Robert
Kingsport, TN 37664 (US)**
• **SHARPE, Robert, Jacks
Madison, AL 35756 (US)**

(74) Representative: **Ricker, Mathias
Wallinger Ricker Schlotter Tostmann
Patent- und Rechtsanwälte Partnerschaft mbB
Zweibrückenstraße 5-7
80331 München (DE)**

(56) References cited:
**GB-A- 204 916     US-A- 5 298 530**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**C07C 67/60, C07C 69/82**

**Description**

**FIELD OF THE INVENTION**

The present invention generally relates to the field of polyester recycle processes and more particularly to polyester recycle process that include depolymerization of polyester via methanolysis, recovery/use of the methanolysis reaction products and conversion of those products to useful chemical compounds.

**BACKGROUND OF THE INVENTION**

Worldwide, polyesters are produced in large quantities well in excess of 75 million tons per year. This level of commercial success is likely attributable in part to polyesters' attractive combination of relative cost, manufacturability and competitive performance attributes. Polyester's physical, chemical and thermal properties make them useful and desirable for a wide variety of end-use applications including clothing, carpeting, and films. Polyethylene terephthalate (PET) is probably one of the most popular types of polyester for many end-uses including one-time use applications such as beverage containers. With the continuing commercial success of polyesters generally and PET specifically has come efforts to recover materials from post-consumer, post-industrial, scrap and other sources and re-use those materials as an alternative to basic disposal methods such as landfills.

In one known recycle method, recycled PET is blended with virgin materials. This approach has been used, for example, to prepare blends of virgin poly(butylene terephthalate) ("PBT") with recycled PET to yield a PBT-based product with recycle content (see, for example, U.S. Patent Published Patent Application No. 2009/0275698). Such blends, however, can be generally immiscible and produce a material that is relatively opaque. Blending, therefore, is not a uniformly satisfactory method to provide commercially valuable end products with recycle content.

In another recycle method, polyesters are depolymerized to form the monomer units originally used in its manufacture. One commercially utilized method for polyester depolymerization is methanolysis. In methanolysis, the polyester is reacted with methanol to produce a depolymerized polyester mixture comprising polyester oligomers, dimethyl terephthalate ("DMT"), and ethylene glycol ("EG"). Other monomers such as, for example, 1,4-cyclohexanedimethanol ("CHDM") and diethylene glycol ("DEG") may also be produced depending on the composition of the polyester in the methanolysis feed stream. Some representative methods for the methanolysis of PET are described in U.S. Pat. Nos. 3,037,050; 3,321,510; 3,776,945; 5,051,528; 5,298,530; 5,414,022; 5,432,203; 5,576,456 and 6,262,294. A representative methanolysis process is also illustrated in U.S. Pat. No. 5,298,530, assigned to the assignee of the present invention. The '530 patent describes a process for the recovery of ethylene glycol and dimethyl terephthalate from scrap polyester. The process includes the steps of dissolving scrap polyester in oligomers of ethylene glycol and terephthalic acid or dimethyl terephthalate and passing super-heated methanol through this mixture. The oligomers can comprise any low molecular weight polyester polymer of the same composition as that of the scrap material being employed as the starting component such that the scrap polymer will dissolve in the low molecular weight oligomer. The dimethyl terephthalate and the ethylene glycol are recovered from the methanol vapor stream that issues from depolymerization reactor.

As known in the art and described for example in U.S. Patent No. 3,448,298, the methanolysis of PET (as well as the preparation of PET from a glycol ester and a dibasic acid) is a reversible, equilibrium reaction wherein, based on measured reaction equilibrium data, the forward reaction is not favored compared to the reverse reaction. At an industrial scale, this represents a significant problem since this generally results in increased energy and/or material usage to achieve high overall yields of DMT from PET. In certain prior art attempts to address this issue, as described for example in U.S. Patent No. 5,298,530, it is proposed to perform methanolysis depolymerization of polyester using a large stoichiometric excess of methanol in superheated vapor form.

Such an approach has a number of drawbacks. For example, the energy usage associated with superheating and/or condensing the excess methanol may markedly increase the operational cost of the process. Potential operational and system equipment cost increases may also apply if the methanol vapor is used to evaporate the DMT and EG products, as the mixture of DMT, EG and methanol will need to undergo one or more separation steps to isolate each of the individual compounds.

A continuing unmet need therefore exists for a polyester methanolysis depolymerization process with increased polyester depolymerization and/or DMT yield and reduced methanol requirements.

**SUMMARY OF THE INVENTION**

In a first aspect, the present invention relates to a method for depolymerizing a polyester. The method of the present invention, in this aspect, includes the steps of (a) treating said polyester with methanol in a depolymerization reaction system comprising at least one depolymerization reactor under conditions sufficient to depolymerize at least some of said polyester and form a depolymerization reaction product comprising dimethyl terephthalate and at least one diol; and (b) converting within said depolymerization reaction system at least some of said diol of said depolymerization reaction product to a compound substantially non-reactive with the dimethyl terephthalate and the polyester.

In another aspect, the present invention relates to a method for producing a cyclic carbonate. The method of the present invention, in this aspect, includes the steps of (a) feeding to a depolymerization system comprising at least one depolymerization reactor a polyester, methanol and a cyclic carbonate-forming reactant; (b) depolymerizing within said depolymerization system at least some of said polyester by reaction with methanol to form dimethyl terephthalate and a cyclic carbonate-forming diol; and (c) reacting within said depolymerization system said cyclic carbonate-forming diol and said cyclic carbonate-forming reactant to form said cyclic carbonate.

Further aspects of the invention are as disclosed and claimed herein.

## BRIEF DESCRIPTION OF THE DRAWINGS

FIG 1 is a schematic representation of an illustrative embodiment of the method of the present invention in its various aspects; and

FIG 2 is a schematic representation of another illustrative embodiment of the method of the present invention in its various aspects.

## DETAILED DESCRIPTION

The term "polyester" as used herein is meant to generally include without limitation homopolyesters as well as copolyesters, terpolyesters and the like and are typically prepared by reacting a difunctional carboxylic acid or its ester, often a dicarboxylic acid, or mixtures of such acids or esters, with a difunctional hydroxyl compound, often a diol or glycol, or mixtures of such diols or glycols. The term "polyester" may also include oligomers and monomers of homopolyesters as well as copolyesters, terpolyesters and the like. The term polyester may also include the diester on monoester form of said polyesters. For example, the diester of poly(ethylene) terephthalate (PET) is bis(2-hydroxyethyl) terephthalate commonly abbreviated as BHET. The difunctional carboxylic acid may be a hydroxy carboxylic acid and the difunctional hydroxyl compound may be an aromatic nucleus bearing 2 hydroxyl substituents such as, for example, hydroquinone.

In a first aspect, the present invention is directed to a method for depolymerization of polyester. The method of this aspect of the present invention includes (a) treating the polyester with methanol in a depolymerization reaction system comprising at least one depolymerization reactor under conditions sufficient to depolymerize at least some of the polyester and form a depolymerization reaction product comprising dimethyl terephthalate and at least one diol; and (b) converting within said depolymerization reaction system at least some of the diol of the depolymerization reaction product to a compound substantially non-reactive with the dimethyl terephthalate and the polyester. In one or more embodiments, the polyester is flowable polyester. "Flowable", as used herein, in intended to include for example melts, solutions, flowable pastes, dispersions and the like wherein a material, component of a mixture or a mixture may be partially or substantially completely melted, partially dissolved or substantially completely dissolved in a solvent or plurality of solvents. As used herein, the phrase "a compound substantially non-reactive with" is intended to mean a compound or plurality of compounds that either reacts minimally or not at all with dimethyl terephthalate and polyester to an extent such that the overall aromatic moieties comprising the dimethyl terephthalate and polyester remain as either dimethyl terephthalate or the original polyester. Non-limiting examples of compounds substantially non-reactive with dimethyl terephthalate and polyester include carbonates, cyclic carbonates and 2,2-dimethyl-1,3-dioxane.

One of ordinary skill will be appreciate that the identity of the at least one diol and the number of diols formed in treating step (a) will vary depending in part on the polyester that is depolymerized in the treating step (a) and may include for example ethylene glycol, diethylene glycol, polyethylene glycol, polytetramethylene glycol neopentyl glycol, p-xylene glycol, 1,2-propanediol, 1,3-propanediol, 1,4-butanediol, 1,5-pentanediol, 1,6-hexanediol, 2,2,4,4-tetramethyl-1,3-cyclobutanediol (TMCD), 1,4-cyclohexanedimethanol (CHDM) and isomers and combinations thereof. In one or more embodiments, the diol is selected from the group consisting of ethylene glycol, 1,3-propanediol, trimethylene glycol, neopentyl glycol, 1,4-butanediol, 1,5- pentanediol, 1,6-hexanediol and isomers and combinations thereof.

In one or more embodiments, treating step (a) includes treating the polyester with methanol. In one or more embodiments, the treating step (a) includes treating said polyester with a stoichiometric excess of methanol. Preferably, the total amount of methanol for treating step (a) is between 2 and 12 equivalent moles per mole of the polyester. As described below, in one or more embodiments methanol is formed in converting step (b) and in some embodiments that methanol formed in converting step (b) may be used to treat polyester in treating step (a). Accordingly, in one or more embodiments, the methanol of treating step (a) includes methanol formed in converting step (b).

In one or more embodiments, the treating step (a) is performed in a solvent or a plurality of solvents in which the polyester may be solubilized. Suitable solvents include, by way of non-limiting example, dimethyl sulfoxide (DMSO), N-methyl-2-pyrrolidone (NMP), dimethyformamide (DMF), dichloromethane, tetrahydrofuran (THF), trifluoroacetic acid (TFA), benzene and xylene. In addition to the temperature and pressure ranges set forth elsewhere herein, other conditions for treating step (a) will be apparent to a person of ordinary skill in the art and are exemplified in U.S. Patent No. 6,136,869.

In one or more embodiments, the converting step (b) includes converting the diol to a carbonate. In one or more embodiments, the carbonate is a cyclic carbonate and the converting step (b) includes converting the diol to a cyclic carbonate. In one or more embodiments, the diol is a cyclic carbonate-forming diol and converting step (b) includes converting the at least one cyclic carbonate-forming diol to a cyclic carbonate. The phrase "cyclic carbonate-forming diol" is intended to include diols that readily react with cyclic carbonate-forming reactants such as dimethyl carbonate, diethyl carbonate, phosgene or urea to form a cyclic carbonate. Similarly, a "cyclic carbonate" is intended to include carbonates formed by the reaction of a cyclic carbonate-forming diol and cyclic carbonate-forming reactants such as dimethyl carbonate, diethyl carbonate, phosgene or urea. Non-limiting examples of cyclic carbonate-forming diols include ethylene glycol, 1,3-propanediol, trimethylene glycol, neopentyl glycol, 1,4-butanediol, 1,5-pentanediol, 1,6-hexanediol and isomers and combinations thereof. Accordingly, cyclic carbonates may include for example trimethylene glycol carbonate, neopentyl glycol carbonate, 1,4-butanediol carbonate and dimeric carbonates of pentanediol or of hexanediol.

In one or more embodiments, converting step (b) comprises reacting said diol with at least one cyclic carbonate-forming reactant selected from the group consisting of dimethyl carbonate, diethyl carbonate, phosgene and urea to form a cyclic carbonate. In one or more embodiments, the cyclic carbonate-forming reactant is a flowable reactant. "Flowable", as used herein, in intended to include for example melts, solutions, flowable pastes, dispersions and the like wherein a material, component of a mixture or a mixture may be partially or substantially completely melted, partially dissolved or substantially completely dissolved in a solvent or plurality of solvents. In one or more embodiments, the converting step (b) includes reacting ethylene glycol with dimethyl carbonate, preferably flowable dimethyl carbonate, to form ethylene carbonate and methanol. In one or more embodiments, the converting step (b) includes reacting ethylene glycol with urea to form ethylene carbonate and ammonia. In one or more embodiments, the converting step (b) includes reacting ethylene glycol with 2,2-dimethoxypropane to form 2,2-dimethyl-1,3-dioxane. In one or more embodiments, the converting step (b) includes reacting ethylene glycol with phosgene to form ethylene carbonate and hydrogen chloride. In one or more embodiments, the converting step (b) includes reacting 1,3-propanediol with dimethyl carbonate to form trimethylene carbonate and methanol.

In one or more embodiments, the treating step (a) and the converting step (b) are performed in the presence of a catalyst. In one or more embodiments, the catalyst is at least one basic compound. In one or more embodiments, the treating step (a) and said converting step (b) are performed in the presence of the same catalyst or the same catalyst load. Suitable catalysts may vary and may be selected based on a number of factors, including reactor design, number of reactors, reaction conditions and the like. Non-limiting examples of useful catalysts include potassium carbonate; calcium oxide; sodium methylate; sodium hydroxide; sodium carbonate; sodium acetate; 1,8-diazabicyclo-[5,4,0]-undec-7-ene (DBU); 1,1,3,3-tetramethylguanadine (TMG); 1,57-triazabicyclo-[4,4,0]-dec-5-ene (TBD); 7-methyl-1,57-triazabicyclo-[4,4,0]-dec-5-ene (MTBD), 1,5-diazabicyclo[4,3,0]non-5-ene (DBN); quinuclidine; 2,2,6,6-tetramethylpiperidine (TMP); pempidine (PMP); tributylamine; triethylamine; 1,4-diazabicyclo-[2,2,2]-octane (DABCO); collidine; 4-(N,N-dimethylamino) pyridine (DMAP); N-methylimidazole (NMI); N,N-dimethylaniline (DMA); hydrotalcite; and combinations thereof.

The treating step (a) and the converting step (b) are performed within a depolymerization reaction system that includes at least one depolymerization reactor. Treating step (a) and converting step (b) form in the depolymerization reaction system a reaction product mixture that includes dimethyl terephthalate; at least one diol; and a compound substantially non-reactive with the dimethyl terephthalate and the polyester. It will be appreciated that the amounts of the reaction product mixture components will vary both as a general matter and with time. For example, the amount of diol in the reaction product mixture generated in treating step (a) may decrease over time, and possibly go to zero, as the diol formed in treating step (a) may be converted in converting step (b) to form the compound substantially non-reactive with the dimethyl terephthalate and the polyester. The reaction product mixture may also include residual polyester that is not depolymerized in the treating step (a). In one or more embodiments, the reaction product mixture is a flowable reaction product mixture.

In one or more embodiments, treating step (a) and converting step (b) are performed concurrently. As used herein, the term "concurrently" is intended to connote that the steps are performed at substantially the same time. In one or more embodiments, treating step (a) and converting step (b) are performed in the presence of the same catalyst load; In one or more embodiments, treating step (a) and converting step (b) are performed under substantially the same temperature and pressure. In one or more embodiments, the treating step (a) and the converting step (b) are performed at a temperature between 105°C and 300°C and a pressure of between 14 psia and 5000 (0,965 and 345 bar). In one or embodiments, the treating step (a) and the converting step (b), are performed at a temperature between 150°C and 300°C and a pressure between 200 psia and 600 (13,8 and 41,4 bar).

In one or more embodiments, the method of present invention includes a step of removing at least some dimethyl terephthalate from the reaction product mixture. As used herein, the term "removing" is intended to include all steps which can render the dimethyl terephthalate unavailable for further reaction with other components of the reaction product mixture. In one or more embodiments, the removing step may include physically separating dimethyl terephthalate from the reaction product mixture. In one or more embodiments, wherein at least some of the dimethyl terephthalate may be flowable dimethyl terephthalate in a flowable reaction product mixture, the removing step may include vaporizing the dimethyl terephthalate, for example by heating the reaction product mixture; lowering the pressure to effect evaporation; introducing a stripping agent such as a low boiling solvent; adding a recyclable reactant such as methanol or dimethyl carbonate, or a combination thereof. In one or more embodiments, wherein at least some of the dimethyl terephthalate may be flowable dimethyl terephthalate in a flowable reaction product mixture, the removing step may include forming solid dimethyl terephthalate from said flowable dimethyl terephthalate, for example by cooling the flowable reaction product mixture an amount to sufficient to solidify molten or dissolved dimethyl terephthalate. The cooling step may include cooling the flowable reaction product mixture to a temperature of about 144°C or less. One potential beneficial consequence of cooling the reaction product mixture or removing the presence of dimethyl terephthalate from the liquid phase may be to further shift the reaction equilibrium in favor of the forward reaction and increasing the conversion of the polyester reactant to dimethyl terephthalate.

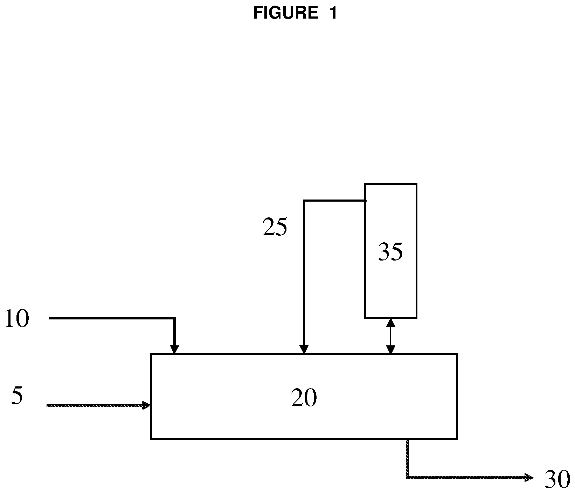

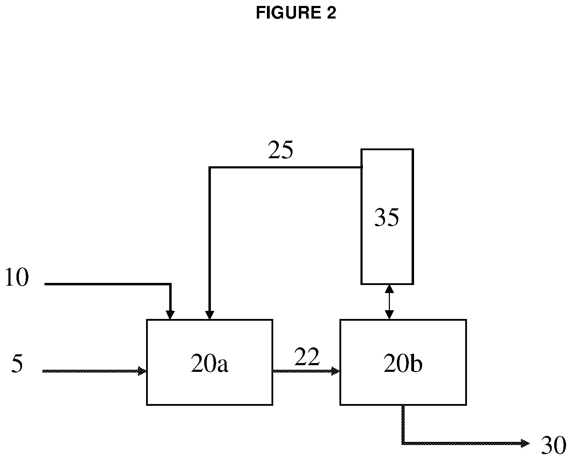

Systems and equipment configurations for practicing the method of the present invention, as well as details and features thereof, may vary. Non-limiting examples for one or more illustrative embodiments of the present invention are depicted in FIGs 1 and 2, which are now described in more detail using, by way of illustration only, a non-limiting example of ethylene carbonate formation by reaction of dimethyl carbonate with ethylene glycol generated by depolymerization of polyester. As depicted in FIG.1, a polyester feed stream 5 and a combined methanol/dimethyl carbonate feed stream 10 may be fed into a depolymerization system that includes depolymerization reactor 20. Though feed stream 10 is shown as a combined methanol/dimethyl carbonate feed stream, is will be appreciated that methanol and dimethyl carbonate may also be fed as separate feed streams and that any combination and number of feed streams may be contemplated to feed polyester, methanol and dimethyl carbonate to the depolymerization system. One or both of the feed streams 5 and 10 may also include catalyst. The treating step (a) and the converting step (b) may be performed in depolymerization reactor 20 to form a flowable reaction product mixture that may include dimethyl terephthalate, ethylene glycol, ethylene carbonate, methanol and residual polyester and dimethyl carbonate. The flowable reaction product mixture may be separated into a methanol-rich stream 25 and an ethylene carbonate-containing stream 30, for example with distillation column 35. At least part of the methanol-rich stream 25 may be returned to depolymerization reactor 20 stream to provide methanol for use in the treating step (a).

As depicted in FIG 2, a polyester feed stream 5 and a combined methanol/dimethyl carbonate feed stream 10 may be fed into a depolymerization system that includes a first depolymerization reactor 20a and a second depolymerization reactor 20b. Though two reactors 20a and 20b are shown for convenience, it will be appreciated that the depolymerization system may include a plurality of depolymerization reactors. Though feed stream 10 is shown as a combined methanol/dimethyl carbonate feed stream, is will be appreciated that methanol and dimethyl carbonate may also be fed as separate feed streams. One or both of the feed streams 5 and 10 may also include catalyst. The treating step (a) and the converting step (b) may be performed in first polymerization reactor 20a to form a first flowable reaction product mixture that may include dimethyl terephthalate, ethylene glycol, ethylene carbonate, methanol and residual polyester and dimethyl carbonate. The first flowable reaction product mixture may be transferred via transfer line 22 to second depolymerization reactor 20b wherein a second flowable reaction product mixture with higher dimethyl terephthalate content as compared to first flowable reaction mixture is formed. The second flowable reaction product mixture may be separated into a methanol-rich stream 25 and an ethylene carbonate-containing stream 30, for example with distillation column 35. At least part of the methanol-rich stream 25 may be returned to another depolymerization reactor 20 stream to provide methanol for the treating step (a).

As described above, the method of the present in one or more embodiments generates a cyclic carbonate as a product. Accordingly, the present invention, in a second aspect, is directed to a method for producing a cyclic carbonate The method of the present invention, in this aspect, includes the steps of (a) feeding to a depolymerization system comprising at least one depolymerization reactor a polyester, methanol and a reactant selected from the group consisting of dimethyl carbonate, diethyl carbonate, phosgene and urea; (b) depolymerizing within said depolymerization system at least some of said polyester by reaction with methanol to form dimethyl terephthalate and a diol; and (c) reacting within said depolymerization system said diol and said reactant to form the cyclic carbonate. Though this aspect of the present invention is characterized here as a method for producing ethylene carbonate, one of ordinary skill will appreciate that it could also be characterized as a method for depolymerization of polyester, in part as its steps include polyester depolymerization. Accordingly, it should be understood by a person of ordinary skill that features and elements described in conjunction with of the one aspect of the present invention, including but not limited to number of depolymerization reactors, depolymerization and chemical reaction conditions such as temperature and pressure, use of catalysts, reactants, reaction products, physical forms of reactants and reaction products and the like are applicable to and useful to describe those features and elements of other aspects.

In one or more embodiments, the reacting step (c) includes reacting ethylene glycol with dimethyl carbonate, preferably flowable dimethyl carbonate, to form ethylene carbonate and methanol. In one or more embodiments, the reacting step (c) includes reacting 1,3-propanediol with dimethyl carbonate to form trimethylene carbonate and methanol. In one or more embodiments, the reacting step (c) includes reacting ethylene glycol with urea to form ethylene carbonate and ammonia. In one or more embodiments, the reacting step (c) includes reacting ethylene glycol with phosgene to form ethylene carbonate and hydrogen chloride.

In one or more embodiments wherein the depolymerization system includes a plurality of depolymerization reactors such as exemplified in FIGs 1 and 2, the method of the present invention may further include the steps of removing from a depolymerization reactor a methanol-rich stream comprising unreacted methanol; separating at least some of the unreacted methanol from the stream; and recycling at least some of the unreacted methanol to another depolymerization reactor. In one or more embodiments wherein the depolymerization system includes a plurality of depolymerization reactors such as exemplified in FIGs 1 and 2, the method of the present invention may further include the steps of removing from a depolymerization reactor a stream comprising unreacted carbonate-forming reactant selected from the group consisting of dimethyl carbonate, diethyl carbonate, phosgene and urea; separating at least some of the unreacted cyclic carbonate-forming reactant from the stream; and recycling at least some of the unreacted cyclic carbonate-forming reactant to another depolymerization reactor.

## Claims

1. A method for depolymerization of polyester, said method comprising:

   (a) treating said polyester with methanol in a depolymerization reaction system comprising at least one depolymerization reactor under conditions sufficient to depolymerize at least some of said polyester and form a depolymerization reaction product comprising dimethyl terephthalate and at least one diol; and
   (b) converting in said depolymerization reaction system at least some of said diol of said depolymerization reaction product to a compound substantially non-reactive with said dimethyl terephthalate and said polyester,

   optionally wherein treating step (a) and converting step (b) form in said depolymerization reaction system a reaction product mixture that includes dimethyl terephthalate; at least one diol; and a compound substantially non-reactive with the dimethyl terephthalate and the polyester.

2. The method of claim 1 wherein said diol is selected from the group consisting of ethylene glycol, 1,3-propanediol, trimethylene glycol, neopentyl glycol, 1,4-butanediol, 1,5-pentanediol, 1,6-hexanediol and isomers and combinations thereof.

3. The method of claim 2 wherein said converting step (b) comprises converting said diol to a carbonate.

4. The method of claim 3 wherein said converting step (b) comprises reacting said diol with at least one cyclic carbonate-forming reactant selected from the group consisting of dimethyl carbonate, diethyl carbonate, phosgene and urea to form a cyclic carbonate.

5. The method of claim 4 wherein said converting step (b) comprises reacting said diol with dimethyl carbonate to form a cyclic carbonate and methanol.

6. The method of claim 5 wherein said converting step (b) comprises reacting 1,3-propanediol with dimethyl carbonate to form trimethylene carbonate and methanol; or wherein said converting step (b) comprises reacting ethylene glycol with dimethyl carbonate to form ethylene carbonate and methanol.

7. The method of claim 5 further comprising a step of using methanol formed in converting step (b) in treating step (a), optionally wherein the molar amount of methanol in said treating step (a) and the molar amount of said dimethyl carbonate in said converting step (b) satisfy the equation

$$A = 2 \times B + C$$

wherein A = total equivalent moles of methanol present per mole of polyester; B= moles of dimethyl carbonate present per mole of polyester; and C = moles of methanol feed per mole of polyester; with B>0 and A >2.

8. The method of claim 1 wherein said treating step (a) and said converting step (b) are performed concurrently.

9. The method of claim 1 wherein said treating step (a) and said converting step (b) are performed in the presence of a catalyst, optionally wherein said catalyst is selected from the group consisting of potassium carbonate, lithium carbonate; cesium carbonate; potassium hydroxide; triethylamine calcium oxide, sodium methylate; potassium methylate; magnesium methylate; magnesium hydroxide, potassium acetate; sodium hydroxide; sodium carbonate;

sodium acetate; 1,8-diazabicyclo-[5,4,0]-undec-7-ene (DBU); 1,1,3,3-tetramethylguanadine (TMG); 1,57-triazabicyclo-[4,4,0]-dec-5-ene (TBD); 7-methyl-1,57-triazabicyclo-[4,4,0]-dec-5-ene (MTBD), 1,5-diazabicyclo[4,3,0] non-5-ene (DBN); quinuclidine; 2,2,6,6-tetramethylpiperidine (TMP); pempidine (PMP); tributylamine; triethylamine; 1,4-diazabicyclo-[2,2,2]-octane (DABCO); collidine; 4-(N,N-dimethylamino) pyridine (DMAP); N-methylimidazole (NMI); N,N-dimethylaniline (DMA); hydrotalcite; potassium tert-butylate; methanesulfonic acid; 4-toluenesulfonic acid; and combinations thereof.

10. The method of claim 4 wherein said converting step (b) comprises reacting said diol with flowable dimethyl carbonate.

11. The method of claim 4 wherein said treating step (a) and said converting step (b) are performed at a temperature between 105°C and 300°C and a pressure of between 0.965 bar (14 psia) and 345 bar (5000 psia); or wherein said treating step (a) and converting step (b) are performed at a temperature between 150°C and 300°C and a pressure between 13.8 bar (200) psi and 41.4 bar (600 psia).

12. The method of claim 1 further comprising the step of removing said dimethyl terephthalate from said depolymerization reaction product, optionally wherein said dimethyl terephthalate is flowable dimethyl terephthalate and said removing step comprises forming solid dimethyl terephthalate from said flowable dimethyl terephthalate; or wherein said dimethyl terephthalate is flowable and the said removing step comprises a step of forming dimethyl terephthalate vapor from said flowable dimethyl terephthalate.

13. The method of claim 1 where said polyester is flowable polyester.

14. A method for producing a cyclic carbonate, said method comprising:

(a) feeding to a depolymerization system comprising at least one depolymerization reactor a polyester, methanol and a cyclic carbonate-forming reactant selected from the group consisting of dimethyl carbonate, diethyl carbonate, phosgene and urea;
(b) depolymerizing within said depolymerization system at least some of said polyester by reaction with methanol to form dimethyl terephthalate and a cyclic carbonate-forming diol selected from the group consisting of ethylene glycol, 1,3-propanediol, trimethylene glycol, neopentyl glycol, 1,4-butanediol, 1,5-pentanediol, 1,6-hexanediol and isomers and combinations thereof;

and
(c) reacting within said depolymerization system said cyclic carbonate-forming diol and said cyclic carbonate-forming reactant to form said cyclic carbonate.

15. The method of claim 2 wherein said converting step (b) comprises reacting said diol with urea to form a cyclic carbonate; or wherein said converting step (b) comprises reacting said diol with phosgene to form a cyclic carbonate.

16. The method of claim 1 wherein said converting step (b) comprises reacting ethylene glycol with 2,2-dimethoxypropane to form 2,2-dimethyl-1,3-dioxane.

**Patentansprüche**

1. Ein Verfahren zur Depolymerisation von Polyester, besagtes Verfahren aufweisend:

(a) Behandeln des besagten Polyesters mit Methanol in einem Depolymerisationsreaktionssystem aufweisend mindestens einen Depolymerisationsreaktor bei Bedingungen geeignet zum Depolymerisieren von mindestens Teilen des besagten Polyesters und zum Bilden eines Depolymerisationsreaktionsproduktes aufweisend Dimethylterephthalat und mindestens einem Diol; und
(b) Umsetzen von mindestens einem Teil des besagten Diols des besagten Depolymerisationsreaktionsproduktes in besagtem Depolymerisationsreaktionssystem zu einer Verbindung im Wesentlichen nicht-reaktiv mit besagtem Dimethylterephthalat und besagtem Polyester,

wobei optional besagter Behandlungsschritt (a) und Umsetzungsschritt (b) in besagtem Depolymerisationsreaktionssystem eine Reaktionsproduktmischung bilden, welche Dimethylterephthalat; mindestens ein Diol; und eine Verbindung im Wesentlichen nicht-reaktiv mit dem Dimethylterephthalat und dem Polyester umfasst.

2. Verfahren nach Anspruch 1, wobei besagtes Diol ausgewählt ist aus der Gruppe bestehend aus Ethylenglykol, 1,3-Propandiol, Trimethylenglykol, Neopentylglykol, 1,4-Butandiol, 1,5-Pentandiol, 1,6-Hexandiol und Isomeren und Kombinationen davon.

3. Verfahren nach Anspruch 2, wobei besagter Umsetzungsschritt (b) das Umsetzen des besagten Diols in ein Carbonat aufweist.

4. Verfahren nach Anspruch 3, wobei besagter Umsetzungsschritt (b) das Reagieren des besagten Diols mit mindestens einem zyklischen Carbonat-bildenden Reagenz ausgewählt aus der Gruppe bestehend aus Dimethylcarbonat, Diethylcarbonat, Phosgen und Harnstoff zum Bilden eines zyklischen Carbonats aufweist.

5. Verfahren nach Anspruch 4, wobei besagter Umsetzungsschritt (b) das Reagieren des besagten Diols mit Dimethylcarbonat zum Bilden eines zyklischen Carbonats und Methanol aufweist.

6. Verfahren nach Anspruch 5, wobei besagter Umsetzungsschritt (b) das Reagieren von 1,3-Propandiol mit Dimethylcarbonat zum Bilden von Trimethylencarbonat und Methanol aufweist; oder wobei besagter Umsetzungsschritt (b) das Reagieren von Ethylenglykol mit Dimethylcarbonat zum Bilden von Ethylencarbonat und Methanol aufweist.

7. Verfahren nach Anspruch 5, weiterhin aufweisend einen Schritt zur Verwendung von Methanol gebildet im Umsetzungsschritt (b) in Behandlungsschritt (a), optional wobei die molare Menge des besagten Dimethylcarbonats in besagtem Umsetzungsschritt (b) die Gleichung

$$A = 2 \times B + C$$

erfüllt, wobei A = die Gesamtäquivalentmol des Methanols vorhanden pro Mol des Polyesters; B = Mol des Dimethylcarbonats vorhanden pro Mol des Polyesters; und C = Mol des Methanols zugeführt pro Mol des Polyesters; mit B>0 und A>2; ist.

8. Verfahren nach Anspruch 1, wobei besagter Behandlungsschritt (a) und besagter Umsetzungsschritt (b) gleichzeitig durchgeführt werden.

9. Verfahren nach Anspruch 1, wobei besagter Behandlungsschritt (a) und besagter Umsetzungsschritt (b) in der Gegenwart eines Katalysators durchgeführt werden, wobei optional besagter Katalysator ausgewählt ist aus der Gruppe bestehend aus Kaliumcarbonat, Lithiumcarbonat; Cäsiumcarbonat; Kaliumhydroxid; Triethylamincalciumoxid, Natriummethylat; Kaliummethylat; Magnesiummethylat; Magnesiumhydroxid, Kaliumacetat; Natriumhydroxid; Natriumcarbonat; Natriumacetat; 1,8-Diazabicyclo-[5.4.0]-undec-7-en (DBU); 1,1,3,3-Tetramethylguanadin (TMG); 1,5,7-Triazabicyclo-[4.4.0]-dec-5-en (TBD); 7-Methyl-1,5,7-triazabicyclo-[4.4.0]-dec-5-en (MTBD), 1,5-Diazabicyclo[4.3.0]non-5-en (DBN); Chinuclidin; 2,2,6,6-Tetramethylpiperidin (TMP); Pempidin (PMP); Tributylamin; Triethylamin; 1,4-Diazabicyclo-[2,2,2]-octan (DABCO); Collidin; 4-(N,N-Dimethylamino)-Pyridin (DMAP); N-Methylimidazol (NMI); N,N-Dimethylanilin (DMA); Hydrotalcit; Kalium-tert.-butylat; Methansulfonsäure; 4-Toluolsulfonsäure; und Kombinationen daraus.

10. Verfahren nach Anspruch 4, wobei besagter Umsetzungsschritt (b) das Reagieren besagten Diols mit fließfähigem Dimethylcarbonat aufweist.

11. Verfahren nach Anspruch 4, wobei besagter Behandlungsschritt (a) und besagter Umsetzungsschritt (b) bei einer Temperatur zwischen 105 °C und 300°C und einem Druck zwischen 0,965 bar (14 psia) und 345 bar (5000 psia) durchgeführt werden; oder wobei besagter Behandlungsschritt (a) und Umsetzungsschritt (b) bei einer Temperatur zwischen 150°C und 300°C und einem Druck zwischen 13,8 bar (200 psi) und 41,4 bar (600 psia) durchgeführt werden.

12. Verfahren nach Anspruch 1 weiterhin aufweisend den Schritt des Entfernens von besagtem Dimethylterephthalat aus besagtem Depolymerisationsreaktionsprodukt, wobei optional besagtes Dimethylterephthalat fließfähiges Dimethylterephthalat ist und besagter Entfernungsschritt das Bilden von festem Dimethylterephthalat aus besagtem fließfähigem Dimethylterephthalat aufweist; oder wobei besagtes Dimethylterephthalat fließfähig ist und besagter Entfernungsschritt einen Schritt zur Bildung von Dimethylterephthalat Dampf aus besagtem fließfähigem Dimethylterephthalat aufweist.

13. Verfahren nach Anspruch 1, wobei besagtes Polyester ein fließfähiges Polyester ist.

14. Ein Verfahren zum Herstellen eines zyklischen Carbonats, besagtes Verfahren aufweisend:

   (a) Zugeben von einem Polyester, Methanol und einem zyklischen Carbonat-bildenden Reagenzes ausgewählt aus der Gruppe bestehend aus Dimethylcarbonat, Diethylcarbonat, Phosgen und Harnstoff zu einem Depolymerisationssystem aufweisend mindestens einen Depolymerisationsreaktor;
   (b) Depolymerisieren von mindestens Teilen des besagten Polyesters durch Reaktion mit Methanol zum Bilden von Dimethylterephthalat und einem zyklischen Carbonat-bildenden Diol ausgewählt aus der Gruppe bestehend aus Ethylenglycol, 1,3-propandiol, Trimethylenglycol, Neopentylglycol, 1,4-Butandiol, 1,5-Pentandiol, 1,6-Hexandiol und Isomeren und Kombinationen davon, innerhalb des Depolymerisationssystems; und
   (c) Reagieren des besagten zyklischen Carbonat-bildenden Diols und des besagten zyklischen Carbonat-bildenden Reagenzes zum Bilden besagten zyklischen Carbonats innerhalb des besagten Depolymerisationssystems.

15. Verfahren nach Anspruch 2, wobei besagter Umsetzungsschritt (b) das Reagieren des besagten Diols mit Harnstoff zum Bilden eines zyklischen Carbonats aufweist; oder wobei der Umsetzungsschritt (b) das Reagieren des besagten Diols mit Phosgen zum Bilden eines zyklischen Carbonats aufweist.

16. Verfahren nach Anspruch 1, wobei besagter Umsetzungsschritt (b) das Reagieren von Ethylenglycol mit 2,2-Dimethoxypropan zum Bilden von 2,2-Dimethyl-1,3-dioxan aufweist.

**Revendications**

1. Procédé de dépolymérisation de polyester, ledit procédé comprenant :

   (a) le traitement dudit polyester avec du méthanol dans un système réactionnel de dépolymérisation comprenant au moins un réacteur de dépolymérisation dans des conditions suffisantes pour dépolymériser au moins une partie dudit polyester et former un produit de réaction de dépolymérisation comprenant du téréphtalate de diméthyle et au moins un diol ; et
   (b) la conversion dans ledit système réactionnel de dépolymérisation d'au moins une partie dudit diol dudit produit de réaction de dépolymérisation en un composé sensiblement non réactif avec ledit téréphtalate de diméthyle et ledit polyester,

   éventuellement dans lequel l'étape de traitement (a) et l'étape de conversion (b) forment dans ledit système réactionnel de dépolymérisation un mélange de produits de réaction qui comprend du téréphtalate de diméthyle ; au moins un diol ; et un composé sensiblement non réactif avec le téréphtalate de diméthyle et le polyester.

2. Procédé selon la revendication 1, dans lequel ledit diol est choisi parmi le groupe constitué d'éthylène glycol, de 1,3-propanediol, de triméthylène glycol, de néopentyl glycol, de 1,4-butanediol, de 1,5-pentanediol, de 1,6-hexanediol et d'isomères et combinaisons de ceux-ci.

3. Procédé selon la revendication 2, dans lequel ladite étape de conversion (b) comprend la conversion dudit diol en un carbonate.

4. Procédé selon la revendication 3 dans lequel ladite étape de conversion (b) comprend la réaction dudit diol avec au moins un réactif apte à former un carbonate cyclique choisi parmi le groupe constitué de carbonate de diméthyle, de carbonate de diéthyle, de phosgène et d'urée afin de former un carbonate cyclique.

5. Procédé selon la revendication 4, dans lequel ladite étape de conversion (b) comprend la réaction dudit diol avec du carbonate de diméthyle afin de former un carbonate cyclique et du méthanol.

6. Procédé selon la revendication 5, dans lequel ladite étape de conversion (b) comprend la réaction de 1,3-propanediol avec du carbonate de diméthyle afin de former du carbonate de triméthylène et du méthanol ; ou dans lequel ladite étape de conversion (b) comprend la réaction d'éthylène glycol avec du carbonate de diméthyle afin de former du carbonate d'éthylène et du méthanol.

7. Procédé selon la revendication 5 comprenant en outre une étape consistant à utiliser le méthanol formé dans l'étape de conversion (b) dans l'étape de traitement (a), éventuellement dans lequel la quantité molaire de méthanol dans ladite étape de traitement (a) et la quantité molaire dudit carbonate de diméthyle dans ladite étape de conversion (b) satisfont à l'équation suivante

$$A = 2 \times B + C$$

où A = nombre total de moles d'équivalent de méthanol présentes par mole de polyester; B = nombre de moles de carbonate de diméthyle présentes par mole de polyester ; et C = nombre de moles d'alimentation en méthanol par mole de polyester ; avec B > 0 et A > 2.

8. Procédé selon la revendication 1 dans lequel ladite étape de traitement (a) et ladite étape de conversion (b) sont effectuées simultanément.

9. Procédé selon la revendication 1 dans lequel ladite étape de traitement (a) et ladite étape de conversion (b) sont effectuées en présence d'un catalyseur, éventuellement dans lequel ledit catalyseur est choisi parmi le groupe constitué de carbonate de potassium, carbonate de lithium, carbonate de césium ; hydroxyde de potassium ; triéthylamine oxyde de calcium, méthylate de sodium ; méthylate de potassium ; méthylate de magnésium ; hydroxyde de magnésium, acétate de potassium ; hydroxyde de sodium ; carbonate de sodium ; acétate de sodium ; 1,8-diazabicyclo-[5,4,0]-undéc-7-ène (DBU) ; 1,1,3,3-tétraméthylguanadine (TMG) ; 1,57-triazabicyclo-[4,4,0]-déc-5-ène (TBD) ; 7-méthyl-1,57-triazabicyclo-[4,4,0]-déc-5-ène (MTBD), 1,5-diazabicyclo[4,3,0]non-5-ène (DBN) ; quinuclidine ; 2,2,6,6-tétraméthylpipéridine (TMP) ; pempidine (PMP); tributylamine; triéthylamine ; 1,4-diazabicyclo-[2,2,2]-octane (DABCO) ; collidine; 4-(N,N-diméthylamino)pyridine (DMAP) ; N-méthylimidazole (NMI) ; N,N-diméthylaniline (DMA) ; hydrotalcite; tert-butylate de potassium ; acide méthanesulfonique ; acide 4-toluènesulfonique ; et combinaisons de ceux-ci.

10. Procédé selon la revendication 4, dans lequel ladite étape de conversion (b) comprend la réaction dudit diol avec un carbonate de diméthyle fluide.

11. Procédé selon la revendication 4 dans lequel ladite étape de traitement (a) et ladite étape de conversion (b) sont effectuées à une température comprise entre 105 °C et 300 °C et à une pression comprise entre 0,965 bar (14 psia) et 345 bar (5000 psia) ; ou dans lequel ladite étape de traitement (a) et ladite étape de conversion (b) sont effectuées à une température comprise entre 150 °C et 300 °C et à une pression comprise entre 13,8 bar (200 psi) et 41,4 bar (600 psia).

12. Procédé selon la revendication 1 comprenant en outre l'étape d'élimination dudit téréphtalate de diméthyle dudit produit de réaction de dépolymérisation, éventuellement dans lequel ledit téréphtalate de diméthyle est un téréphtalate de diméthyle fluide et ladite étape d'élimination comprend la formation de téréphtalate de diméthyle solide à partir dudit téréphtalate de diméthyle fluide ; ou dans lequel ledit téréphtalate de diméthyle est fluide et ladite étape d'élimination comprend une étape de formation de vapeur de téréphtalate de diméthyle à partir dudit téréphtalate de diméthyle fluide.

13. Procédé selon la revendication 1 dans lequel ledit polyester est un polyester fluide.

14. Procédé de production d'un carbonate cyclique, ledit procédé comprenant :

(a) l'alimentation d'un système de dépolymérisation comprenant au moins un réacteur de dépolymérisation avec un polyester, du méthanol et un réactif apte à former un carbonate cyclique choisi parmi le groupe constitué de carbonate de diméthyle, de carbonate de diéthyle, de phosgène et d'urée ;
(b) la dépolymérisation dans ledit système de dépolymérisation d'au moins une partie dudit polyester par réaction avec le méthanol afin de former du téréphtalate de diméthyle et un diol apte à former un carbonate cyclique choisi parmi le groupe constitué d'éthylène glycol, de 1,3-propanediol, de triméthylène glycol, de néopentyl glycol, de 1,4-butanediol, de 1,5-pentanediol, de 1,6-hexanediol et d'isomères et combinaisons de ceux-ci ; et
(c) la mise en réaction dans ledit système de dépolymérisation dudit diol apte à former un carbonate cyclique et dudit réactif apte à former un carbonate cyclique afin de former ledit carbonate cyclique.

15. Procédé selon la revendication 2, dans lequel ladite étape de conversion (b) comprend la réaction dudit diol avec de l'urée afin de former un carbonate cyclique ; ou dans lequel ladite étape de conversion (b) comprend la réaction dudit diol avec du phosgène afin de former un carbonate cyclique.

16. Procédé selon la revendication 1, dans lequel ladite étape de conversion (b) comprend la réaction d'éthylène glycol avec du 2,2-diméthoxypropane afin de former du 2,2-diméthyl-1,3-dioxane.

**FIGURE  1**

**FIGURE 2**

**EP 4 077 267 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 20090275698 A
- US 3037050 A
- US 3321510 A
- US 3776945 A
- US 5051528 A
- US 5298530 A
- US 5414022 A
- US 5432203 A
- US 5576456 A
- US 6262294 B
- US 3448298 A
- US 6136869 A